# EUROPÄISCHE PATENTSCHRIFT

(11) **EP 1 514 523 B1**
(45) Veröffentlichungstag und Bekanntmachung des Hinweises auf die Patenterteilung: **27.03.2013**
(21) Anmeldenummer: 04090313.0
(22) Anmeldetag: 13.08.2004
(51) Int. Cl.: A61B 18/14, A61B 5/042

(54) **Katheter**
Catheter
Cathéter

(30) Priorität: 11.09.2003 DE 10342709
(43) Veröffentlichungstag der Anmeldung: 16.03.2005
(73) Patentinhaber: BIOTRONIK SE & Co. KG, 12359 Berlin (DE)
(72) Erfinder: Geistert, Wolfgang, Dr., 79618 Rheinfelden (DE)
(74) Vertreter: Lindner-Vogt, Karin L.

(56) Entgegenhaltungen:
- EP-A- 1 008 327
- US-A- 5 281 215
- US-A- 5 755 760

## Beschreibung

Gegenstand der Patentanmeldung ist ein Ablationskatheter zur intravaskulären Applikation, mit einem proximalen und einem distalen Ende und einer Katheterlängsachse, mit einem sich entlang der Katheterlängsachse von dem proximalen bis zu dem distalen Ende erstreckenden Schaft mit einer fluiddichten Schaftwand, welche ein Lumen ausbildet, mit mindestens einer Ablationselektrode am distalen Ende des Schaftes, wobei das Lumen ein erstes und ein zweites Lumen umfasst, welche jeweils ausgebildet sind, ein Fluid zu führen und sich vom proximalen bis zumindest in die Nähe des distalen Endes des Katheters erstrecken und von dem Schaft umschlossen sind, Der Ablationskatheter weist auch mindestens einen elektrisch leitfähigen Draht auf, welcher sich vom proximalen bis zumindest in die Nähe des distalen Endes des Katheters entlang der Katheterlängsachse erstreckt und sich auf mindestens einer zum Einführen in einen Körper vorgesehenen Abschnittslänge des Katheters im zweiten Lumen befindet, wobei zwischen einem in dem zweiten Lumen befindlichen Fluid und dem elektrisch leitfähigen Draht ein geringerer Wärmeübergangswiderstand herrscht, als zwischen dem Draht und der Schaftwand, so dass in dem Draht entstehende Wärme an das im Lumen fließende Fluid wirksam abgeführt werden kann. Weiterhin umfasst ein solcher Ablationskatheter mindestens einen Temperatursensor, welcher angeordnet ist, eine Temperatur zu erfassen, welche im Falle einer Erwärmung des Drahtes zumindest überwiegend von der Erwärmung des Drahtes abhängt.

Aus dem Stand der Technik sind Katheter bekannt, welche Drähte in Form von Steuerdrähten zur Auslenkung des distalen Endes eines Katheters oder elektrischen Zuleitungen für Elektroden, beispielsweise Hochfrequenzleitungen bei Ablationskathetem oder auch in Form von elektrischen Zuleitungen für Sensoren enthalten. Die Drähte in Form von Steuerdrähten oder elektrischen Zuleitungen sind üblicherweise im Inneren eines Katheterschaftes geführt.

Bei diesen aus dem Stand der Technik bekannten Ablationskathetern stellt sich das Problem, dass die im Katheterschaft verlaufenden Drähte im Falle eines Einsatzes des Katheters während einer Magnetresonanztomographie durch hohe, elektromagnetisch induzierte Ströme deutlich erwärmt werden, so dass es durch Wärmeleitung zu einer Erwärmung des Katheterschaftes kommt, welche die Gefahr von Koagulationen entlang des Katheterschaftes mit sich bringt.

Ebenso besteht die Gefahr von Koagulationen entlang des im Körper befindlichen Katheterschaftes durch hohe, in den Zuleitungen zur Ablationselektrode geleitete Hochfrequenzströme, welche zu einer Erwärmung der Zuleitungen und über Wärmeleitung zur Erwärmung des Katheterschaftes führen.

Aus den Dokumenten DE 693 32 414 T2, US 5,755,760 und EP 1 008 327 A2 sind Ablationskatheter bekannt, welche unter anderem zwei Lumina zum Führen eines Kühlmittels und Temperatursensoren am distalen Ende zur Überwachung der Temperatur einer Ablationselektrode und des Kühlmittels aufweist.

Aus der US 2003/0004506 und der US 5,348,554 sind Ablationskatheter bekannt, die zusätzlich zu den soeben genannten Kathetern flüssigkeitsgekühlte Ablationselektroden aufweisen.

Aus der US 5,980,515 ist ein Katheter mit einem rotierenden Sägewerkzeug und einer Spülvorrichtung bekannt, wobei die Spülvorrichtung auch zur Kühlung einer Ablationselektrode dienen kann.

Das Problem der Katheterschafterwärmung durch in dem Schaft verlaufende elektrisch leitfähige Drähte wird in diesen Patentschriften nicht genannt und nicht gelöst.

Die der Erfindung zugrunde liegende Aufgabe besteht darin, einen Katheter anzugeben, der eine effektive Ablationselektrodenkühlung sicherstellt, bei welchem aber auch das zuvor beschriebene Problem der Katheterschafterwärmung durch in dem Schaft verlaufende elektrisch leitfähige Drähte, insbesondere bei Anwendung einer Magnetresonanztomografie nicht auftritt.

Diese Aufgabe wird gemäß Anspruch 1 durch einen Ablationskatheter der eingangs genannten Art gelöst, bei welchem das Lumen ein drittes Lumen umfasst, welches ausgebildet ist, ein Fluid zu führen und sich vom proximalen bis zumindest in die Nähe des distalen Endes des Katheters zu erstreckt und von dem Schaft umschlossen ist und bei dem das distale Ende ausgebildet ist, ausschließlich ein in einem ersten Lumen strömendes Fluid der mindestens einen Ablationselektrode zur Kühlung zuzuführen und ein in dem ersten Lumen zum distalen Ende strömendes Fluid zusammen mit dem Fluid zur Kühlung des mindestens einen Drahtes, welches durch das zweite Lumen strömt, in dem dritten Lumen in Richtung proximales Ende zurückzuführen.

Der Temperatursensor kann mit dem Draht wärmeleitverbunden sein. Unter dem Begriff "wärmeleitverbunden" wird hier sowohl eine Wärmeübertragung über Atomarschwingungen als auch eine Wärmeübertragung durch Massetransport verstanden. Die wärmeleitende Verbindung kann somit sowohl Konvektionsleitung als auch Phononenleitung einschließen.

Der Temperatursensor ist beispielsweise an einem vom distalen Ende beabstandeten Ort angeordnet, eine Temperatur der Schaftwand, aufgrund einer in dem Draht erzeugten Wärme, auf dem zum Einführen in einen Körper vorgesehenen Abschnitt des Schaftes, bei welchem die Schaftwandtemperatur wesentlich von der Drahttemperatur abhängt, direkt oder indirekt zu erfassen.

Zur direkten Erfassung der Schaftwandtemperatur ist der Temperatursensor auf oder in der Schaftwand angeordnet. Mit dieser Anordnung kann die Gefahr der Ausbildung von Koagulationen direkt aus einem Schaftwandtemperaturwert abgeleitet werden.

Zur indirekten Erfassung der Schaftwandtemperatur kann der Temperatursensor auf der Wärmeübertragungsstrecke vom Draht bis zur Schaftwand angeordnet sein.

In einem Beispiel, das nicht Teil der Erfindung ist, kann der Katheter in der Nähe des distalen Endes wenigstens eine Öffnung aufweisen, welche mit wenigstens einem der Lumina fluidverbunden ist und welche derart gestaltet ist, dass Fluid aus dem Katheter nach aussen strömen kann.

In diesem Beispiel, kann der Katheter mit zwei Lumina am distalen Ende wenigstens eine Öffnung aufweisen, über welche das Fluid nach außen strömen kann. Der Katheter weist in diesem Beispiel eine durch die Lumina gebildete Fluidstromführung mit einem Auslass am distalen Ende zur Kühlung der Ablationselektrode und/oder das die Elektrode umgebende Gewebe auf.

Alternativ kann der Katheter dieses Beispiels auch mit einer unidirektionalen Fluidführung ausgeführt sein. Eine solche Öffnung ist bei Ablationskathetern aus dem Stand der Technik bereits bekannt, wobei die Öffnung im Katheter derart angeordnet ist, dass eine an der Ablationselektrode erzeugte Wärme teilweise an das Fluid abgeführt werden kann.

Bei der Ausführungsform ist das distale Ende ausgebildet, ausschließlich ein in einem zweiten Lumen strömendes Fluid der Ablationselektrode zuzuführen. Diese Ausführungsform gestattet vorteilhaft die Realisierung getrennter-Kühlungen mit jeweils unterschiedlicher Temperaturregelung für zu kühlende Drähte und die Ablationselektrode. Der Katheter weist in dieser Ausführungsform drei getrennte Lumina auf. Ein erstes Lumen ist als drahtführendes Lumen ausgebildet, in welchem entlang der Katheterlängsachse zu kühlende Drähte angeordnet sind. Der Temperatursensor ist bei dieser Ausführungsform vorzugsweise in dem drahtführenden Lumen oder in dessen Nähe mit einem guten Wärmekontakt angeordnet. Das distale Ende ist hierbei ausgebildet, das zur Kühlung der Drähte verwendete Fluid über ein drittes Lumen zurückzuführen. Alternativ dazu ist das distale Ende ausgebildet, das zur Kühlung der Ablationselektrode verwendete Fluid entweder über eine Öffnung aus dem Katheter austreten zu lassen oder ebenfalls über das dritte Lumen zurückzuführen. Da in diesem Fall über das in dem dritten Lumen strömende Fluid sowohl die Wärme der Ablationselektrode als auch die Wärme des Drahtes abgeführt wird, ist im Falle einer Anordnung des Temperatursensors außerhalb des drahtführenden Lumens ein größerer Wärmeübergangswiderstand zwischen dem dritten Lumen und dem Temperatursensor vorgesehen als zwischen dem Temperatursensor und dem drahtführenden Lumen.

Weiter bevorzugt befindet sich das zweite Lumen und/oder weitere Lumina innerhalb des ersten Lumens. Die Lumina können durch einzelne Schläuche gebildet sein. Alternativ dazu können die Lumina entlang der Katheterlängsachse durch wenigstens eine Trennwand in einem Schlauch und die Fluidpassage durch wenigstens einen Durchbruch gebildet sein. Denkbar ist auch eine Ausführungsform, welche eine Kombination aus durch Trennwände abgetrennte Lumina und durch Einzelschläuche gebildete Lumina aufweist.Es ergeben sich,drei Varianten für die Realisierung der Lumina in Inneren des Katheters: die Lumina können durch Trennwände voneinander getrennt nebeneinander in einem Schlauch verwirklicht sein. Es können alternativ mehrere Schläuche in einem Hülllumen angeordnet sein. Diese Schläuche können nebeneinender oder ineinander (koaxial) angeordnet sein.

In einer bevorzugten Ausführungsform ist der Draht eine elektrische Zuleitung. Die elektrische Zuleitung kann als ein oder mehradrige Zuleitung ausgebildet sein, beispielsweise als Zuleitung für eine Ablationselektrode oder als Zuleitung für Sensoren.

Der Draht kann auch ein Steuerdraht sein, welcher in dem Inneren des Lumens in Richtung der Katheterlängsachse angeordnet ist. Ein solcher Steuerdraht kann beispielsweise in einem Führungsdraht angeordnet sein. Bevorzugt ist der Steuerdraht ausgebildet, das distale Ende eines Katheters mit einer Richtungskomponente quer zur Katheterlängsachse auszulenken. Das distale Ende des Katheters kann dadurch über den Steuerdraht verkrümmt werden und der Katheter somit innerhalb eines Körperlumens in Kurven geführt werden. Der Steuerdraht ist dazu am distalen Ende des Katheters befestigt und ist in dem Inneren des Lumens in Richtung der Katheterlängsachse bevorzugt verschieblich angeordnet. Im Sinne der Erfindung kann auch ein Führungsdraht mittels eines Lumens derart gestaltet sein, dass er mittels eines Fluids zu kühlen ist. Die Erwärmung eines solchen Führungsdrahtes kann im Falle der Magnetresonanztomographie dadurch erfolgen, dass eine in eine übliche Drahtwendel oder einen Metallschaft eines solchen Führungsdrahtes Ströme induziert werden. Durch ein im Inneren der Drahtwendel angeordnetes Lumen eines Führungsdrahtes ist dieser kühlbar und stellt ebenfalls einen Katheter im Sinne der Erfindung dar. Der per Induktion erwärmte Draht eines solchen Führungsdrahtes ist somit beispielsweise eine Metallwendel oder ein Metallschaft des Führungsdrahtes.

In der Ausführungsform weist der Katheter wenigstens einen Temperatursensor zur Erfassung der Temperatur des Fluides oder des Drahtes oder der Schaftwand auf.

Der Katheter kann auch wenigstens einen Temperatursensor zum Erfassen der Temperatur eines am proximalen Ende des Katheters austretenden Fluides aufweisen.

Ein Temperatursensor kann auch entlang der Katheterlängsachse im Bereich der Mitte des zur intrakorporalen Invasion vorgesehenen Abschnittes des Katheterschaftes angeordnet sein. Das gewährt beispielsweise bei Ablationskathetern einen ausreichenden Abstand von einer Ablationselektrode und der von dieser abgegebenen Wärme.

Ein Schaft eines Katheters kann im Sinne der Erfindung entlang der Katheterlängsachse mehrere Temperatursensoren aufweisen. Die Temperatursensoren sind angeordnet, die Temperatur eines Drahtes und/oder der Schaftwand und/oder eines Lumens zu erfassen. Beispielsweise sind die Temperatursensoren durch mehrere spiralförmig in Umfangsrichtung des Katheterschaftes umlaufende Drähte oder durch entlang der Katheterschaftlängsachse laufende Längsdrähte gebildet, wobei die spiralförmig umlaufenden Drähte und die Längsdrähte ausgebildet sind, sich in einem Magnetfeld eines Magnetresonanztomographen nicht wesentlich zu erwärmen. Dazu sind die Temperatursensoren bevorzugt hochohmig ausgebildet.

In einer Ausführungsvariante ist der Katheter ein Ablationskatheter. Dazu umfasst der Katheter am distalen Ende eine Elektrode, welche mit dem Draht elektrisch verbunden ist und über welche ein hochfrequenter elektrischer Strom an ein in der Umgebung der Elektrode befindliches Gewebe abgegeben werden kann. In dem Gewebe wird dadurch Wärme erzeugt, durch welche das Gewebe geschädigt oder zerstört wird. Mit einem Ablationskatheter kann somit gezielt insbesondere pathologisch verändertes Gewebe zerstört werden, um dadurch beispielsweise die Reizleitung von Herzaktionssignalen lokal zu blockieren.

Der Katheter kann auch ein Mappingkatheter sein. Dazu umfasst der Katheter im Bereich des distalen Endes eine oder mehrere Sensing-Elektroden. Der Katheter kann auch eine Schrittmacher- oder Defibrillationselektrode sein.

In einer bevorzugten Ausführungsform ist der Katheter als Ablations- und Mappingkatheter ausgebildet, bei welchem die Merkmale eines Ablationskatheters und eines Mappingkatheters an genau einem Katheter verwirklicht sind. Dazu umfasst der Katheter neben wenigstens einer Ablationselektrode wenigstens eine Sensingelektrode.

Die Erfindung betrifft auch eine Anordnung zur intravaskulären Invasion, umfassend einen Katheter gemäß einer der vorgenannten Ausführungsformen und eine Fluidzufuhreinheit, welche mit wenigstens einem Lumen des Katheters fluidverbunden und ausgebildet ist, einen Fluidstrom in das Lumen zu erzeugen.

Bevorzugt umfasst die Anordnung auch eine Temperaturkontrolleinheit, welche mit wenigstens einem Temperatursensor des Katheters verbunden ist.

Die Anordnung kann außerdem eine Kühleinheit umfassen, welche mit wenigstens einem Lumen des Katheters fluidverbunden und ausgebildet ist, Wärme aus einem Fluid abzuführen. Vorzugsweise ist die Kühleinheit ausgebildet, in Abhängigkeit von einem Kühlsteuersignal Wärme aus dem Fluid abzuführen. Weitere Vorteile ergeben sich, wenn die Kühleinheit ausgebildet ist, die aus dem Fluid abgeführte Wärmeleistung in Abhängigkeit von dem Steuersignal zu verändern. Die Kühleinheit kann dazu wenigstens ein Peltierelement enthalten. Die Anordnung kann eine Temperaturregeleinrichtung zur Einhaltung einer vorbestimmten Fluidtemperatur, beispielsweise in einem Kühlkreislauf, enthalten.

Die Fluidzufuhreinheit enthält in einer vorteilhaften Ausführungsvariante eine steuerbare Pumpeinheit, welche wenigstens mit einem Lumen des Katheters fluidverbunden und ausgebildet ist, in Abhängigkeit von einem Fluidsteuersignal ein Fluid in das Lumen zu pumpen. Weiter vorteilhaft kann die Pumpeinheit ausgebildet sein, in Abhängigkeit von dem Fluidsteuersignal ein vorbestimmtes Fluidvolumen pro Zeit in das Lumen zu pumpen.

Die Fluidzufuhreinheit kann auch durch eine Anordnung mit einem Fluidbehälter und einer Zuleitung gebildet sein, wobei in Falle einer höher gelegenen Anordnung des Fluidbehälters im Verhältnis zu dem Katheter, wie bei einem Infusionstropf, das Fluid abwärts in den Katheter fließen kann.

In einer Ausführungsvariante weist die Anordnung einen Kühlkreislauf, in welchem die Pumpeinheit und die Kühleinheit enthalten sind, und eine Temperaturkontrolleinheit auf, wobei die Temperaturkontrolleinheit mit wenigstens einem Temperatursensor, der Pumpeinheit oder der Kühleinheit oder beiden wirkverbunden ist. Der Temperatursensor ist ausgebildet, ein eine Temperatur repräsentierendes Temperatursignal zu erzeugen und die Temperaturkontrolleinheit ist ausgebildet, ein von dem Temperatursensor erzeugtes Temperatursignal auszuwerten, und die Pumpeinheit oder die Kühleinheit oder beide gemäß einem in der Temperaturkontrolleinheit vorgegebenen Regelalgorithmus zu steuern und dazu entsprechende Steuersignale zu erzeugen.

Die Erfindung soll nun im Folgenden anhand von Figuren erläutert werden.
- Fig. 1: zeigt schematisch dargestellte Querschnitte eines Katheters.
- Fig. 2: zeigt schematisch Darstellungen zweier Längsschnitte distaler Enden von Ablationskathetern.
- Fig. 3: zeigt - schematisch dargestellt - ein Ausführungsbeispiel einer Anordnung mit einem Ablationskatheter mit gekühlten Drähten.

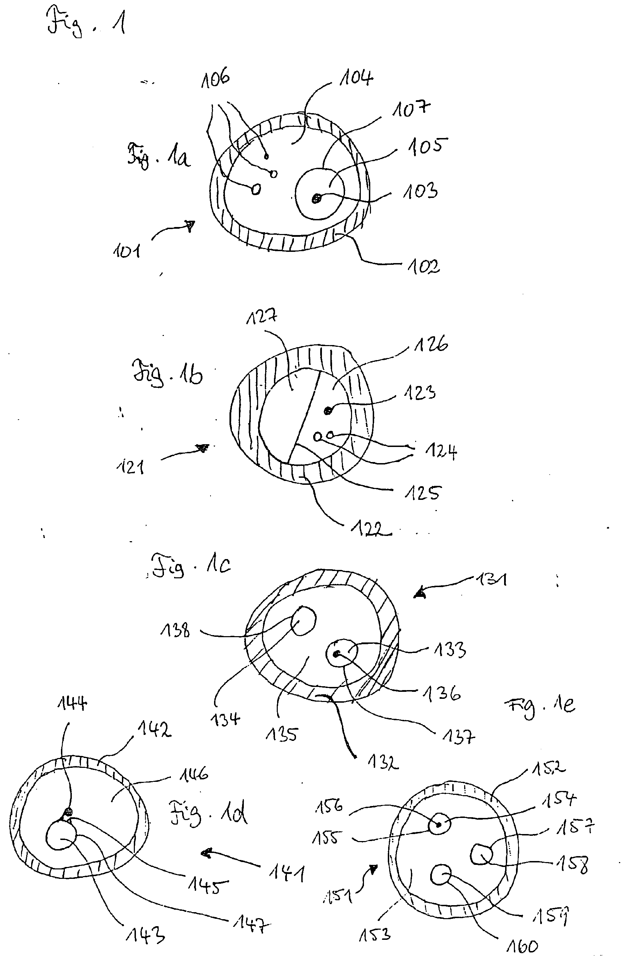

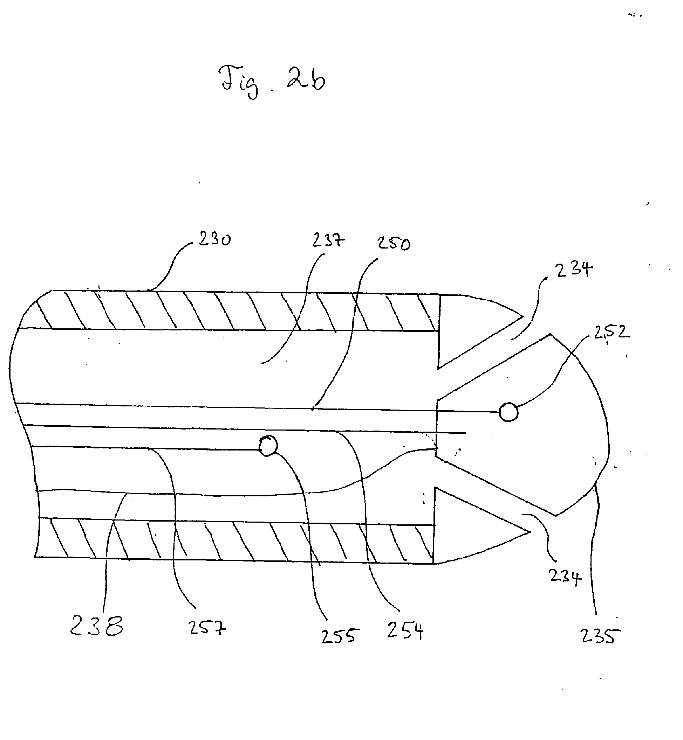

Fig. 1a-1d und 2b stellen Beispiele dar, welche nicht Teil der Erfindung sind.

In Figur 1a ist ein Querschnitt eines Katheters 101 schematisch dargestellt. Der Katheter 101 umfasst einen Katheterschaft mit einer Schaftwand 102 und einem ersten Lumen 104, welches von der Schaftwand 102 umschlossen ist. In dem Lumen, 104 befinden sich Drähte 106, welche als elektrische Zuleitungen ausgebildet sein können. In dem Lumen 104 befindet sich auch ein zweites Lumen 105, welches von einer zweiten Schaftwand 107 umschlossen ist. In dem zweiten Lumen 105 befindet sich ein zu kühlender Draht 103. Die Schaftwände 102 und 107 sind fluiddicht ausgebildet.

Bei einer Anwendung eines Katheters mit einem solchen Katheterschaftquerschnitt kann beispielsweise das zweite Lumen 105 von einem Fluid,. beispielsweise einer Kühlflüssigkeit, durchströmt sein.

Das Durchströmen kann in Form eines unidirektionalen Flusses realisiert sein. Dazu weist der Katheter im Bereich des proximalen Endes einen Eintritt für das Fluid und im Bereich des distalen Endes einen Austritt für das Fluid auf.

Das Durchströmen kann auch in Form eines Gegenstromes realisiert sein. Dazu weist der Katheter im Bereich des distalen Endes eine in dieser Abbildung nicht dargestellte Fluidpassage auf, durch welche die Lumina 105 und 104 im Bereich des distalen Endes fluidverbunden sind. Das Fluid kann nun beispielsweise in dem zweiten Lumen 105 in Richtung distales Ende strömen und durch das erste Lumen 104 über die Fluidpassage zum proximalen Katheterende zurückströmen. Denkbar ist auch eine umgekehrte Flussrichtung, so dass das Fluid in dem ersten Lumen 104 zum distalen Ende strömt und in dem zweiten Lumen 105 zum proximalen Ende zurückströmt.

Figur 1b zeigt einen Querschnitt eines Katheterschaftes 121 mit einer Schaftwand 122, welche ein erstes Lumen 127 und ein zweites Lumen 126 umschließt. Das erste Lumen 127 und das zweite Lumen 126 sind durch eine Trennwand 125 voneinander getrennt. In dem zweiten Lumen 126 befindet sich ein elektrisch leitfähiger Draht 123 und elektrische Zuleitungen 124. Die Schaftwand 122 und die Trennwand 125 sind fluiddicht ausgebildet.

Beispielsweise kann in dem zweiten Lumen ein Fluid zum distalen Ende des Katheters strömen, im Bereich des distalen Endes durch eine nicht dargestellte Fluidpassage in das erste Lumen 127 geleitet werden und dort zum proximalen Ende des Katheters zurückströmen.

Figur 1c zeigt einen Querschnitt eines Katheterschaftes 131 mit einer fluiddichten Schaftwand 132, welche ein Lumen 135 umschließt. Das Lumen 135 kann beispielsweise einen wärmeisolierenden Schaum aufweisen. Das Lumen 135 umfasst ein erstes Lumen 134, welches von einer ersten Schaftwand 138 umschlossen ist. Weiter umfasst das Lumen 135 ein zweites Lumen 133, welches von einer zweiten Schaftwand 137 umschlossen ist und einen Draht 136 aufweist. Beispielsweise können das zweite Lumen 133 und das erste Lumen 134 von einem Fluid durchströmt sein. Das Fluid strömt dabei beispielsweise in den Lumina in jeweils entgegengesetzten Richtungen. Die erste Schaftwand 138 und die zweite Schaftwand 137 sind dazu fluiddicht ausgebildet.

Figur 1d zeigt einen Querschnitt eines Katheterschaftes 141, welcher eine Schaftwand 142 aufweist, welche ein erstes Lumen 146 umschließt. In dem ersten Lumen 146 ist ein zweites Lumen 143 angeordnet, welches von einer zweiten Schaftwand 147 umschlossen ist. Auf der dem ersten Lumen zugewandten Oberfläche der zweiten Schaftwand ist ein Draht 144 mittels eines wärmeleitfähigen Klebers 145 angebracht. Das zweite Lumen 143 kann ein Fluid führen. In dieser Ausführungsform kann das in dem zweiten Lumen befindliche Fluid eine von dem Draht 144 abgegebene Wärme, welche über den wärmeleitfähigen Kleber 145 und die zweite Schaftwand 147 zu dem Fluid geleitet wird, aufnehmen. Dazu ist die zweite Schaftwand 147 fluiddicht und wärmeleitend ausgebildet. Der in dieser Anordnung gebildete Wärmeübergangswiderstand des Lumens 146 von dem Draht 144 zur Schaftwand 142 ist größer ausgebildet, als der Wärmeübergangswiderstand von dem Draht 144 zu dem Lumen 143, gebildet durch den wärmeleitfähigen Kleber 145 und die wärmeleitfähige zweite Schaftwand 147.

Figur 1e zeigt einen Querschnitt eines Katheterschaftes 151 mit einer fluiddichten Schaftwand 152, welche ein Lumen 153 umschließt. Das Lumen 153 kann beispielsweise einen wärmeisolierenden Schaum aufweisen. Das Lumen 153 umfasst ein erstes Lumen 158, welches von einer ersten Schaftwand 157 umschlossen ist. Weiter umfasst das Lumen 153 ein zweites Lumen 154, welches von einer zweiten Schaftwand 155 umschlossen ist und einen Draht 156 aufweist. Das Lumen 153 umfasst weiter ein drittes Lumen 159, welches von einer Schaftwand 160 umschlossen ist. Die drei Lumina 154, 158 und 159 können von einem Fluid durchströmt sein. Das Fluid strömt dabei beispielsweise in dem ersten Lumen 158 und in dem zweiten Lumen 154 in die gleiche Richtung, beispielsweise zum distalen Ende des Katheters, in dem dritten Lumen 159 in dazu entgegengesetzter Richtung, beispielsweise zum proximalen Ende des Katheters. Die Schaftwände 155, 157 und 160 sind dazu fluiddicht ausgebildet.

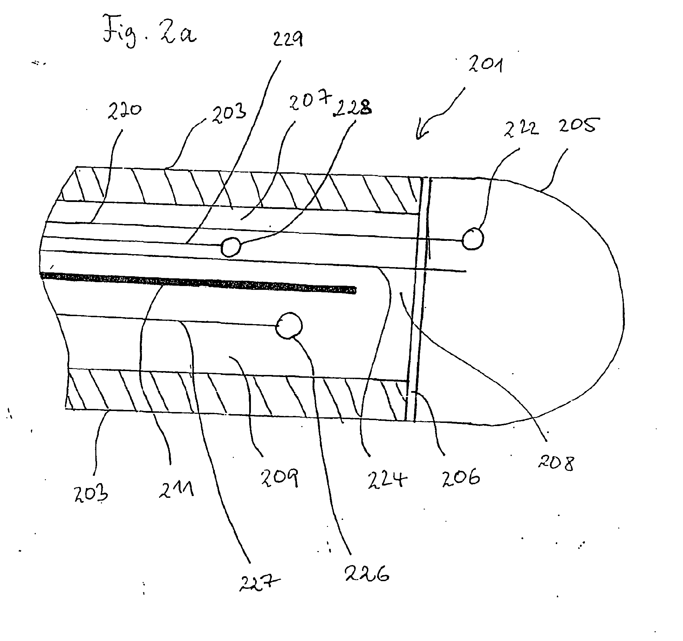

Figur 2a zeigt schematisch einen Längsschnitt des distalen Endes eines Ablationskatheters 201 mit einer sich vom distalen zum proximalen Ende des Katheters erstreckenden Schaftwand 203, welche ein erstes Lumen 209 und ein zweites Lumen 207 umschließt. Die Lumina 209 und 207 sind durch eine entlang der Katheterlängsachse angeordnete Trennwand 211 voneinander getrennt, wobei die Trennwand 211 am distalen Ende des Katheters eine Fluidpassage in Form eines Durchbruchs 208 aufweist. Am äußeren distalen Ende des Katheters ist eine Elektrode 205 angebracht, welche über eine wärmeleitfähige, elektrisch isolierende Schicht 206 mit dem Lumen 207 und mit dem Lumen 209 in Wärmekontakt steht. Die Schaftwand 203, die Trennwand 211 und die Isolierschicht 206 sind fluiddicht ausgebildet. Die Elektrode 205 weist einen Elektrodentemperatursensor 222 auf, welcher mit einer entlang der Katheterlängsachse zum proximalen Ende des Katheters verlaufenden elektrischen Verbindungsleitung 220 verbunden ist, welche in dem zweiten Lumen 207 angeordnet ist. Der Katheter umfasst auch einen Fluidtemperatursensor 226, welcher im Bereich des distalen Endes des Katheters 201 in dem ersten Lumen 209 angeordnet ist und mit einer zum proximalen Ende des Katheters in dem ersten Lumen entlang der Katheterlängsachse verlaufenden elektrischen Verbindungsleitung 227 verbunden ist. Der Katheter umfasst auch im Bereich des distalen Endes einen Drahttemperatursensor 228, welcher mit einer in dem zweiten Lumen zum proximalen Ende des Katheters verlaufenden Verbindungsleitung 229 verbunden ist und in dem zweiten Lumen angeordnet ist.

Wenn ein Fluid in dem zweiten Lumen 207 zum distalen Ende des Katheters strömt und über den Durchbruch 208 und das erste Lumen zum proximalen Ende des Katheters zurückströmt, so wird die durch die Drähte bedingte Erwärmung des Fluides vom Temperatursensor 228, die Temperatur der Elektrode 205 von dem Elektrodentemperatursensor 222 und die Fluidtemperatur des Fluides nach Aufnahme der über die Isolierschicht 206 abgegebenen Wärme des Elektrodenkopfes von dem Temperatursensor 226 erfasst. Der Katheter weist auch einen Zuleitungsdraht 224 auf, welcher in dem zweiten Lumen 204 vom distalen bis zum proximalen Ende des Katheters entlang der Katheterlängsachse geführt ist und am distalen Ende des Katheters befestigt ist. Der Zuleitungsdraht 224 ist elektrisch leitend ausgebildet und ist mit der Elektrode 205 elektrisch verbunden. In diesem Ausführungsbeispiel kann der Elektrode 205 Hochfrequenzenergie über den elektrisch leitenden Zuleitungsdraht 224 zugeführt werden.

Figur 2b zeigt schematisch einen Längsschnitt des distalen Endes eines Ablationskatheters 230 mit einer sich vom distalen zum proximalen Ende des Katheters erstreckenden Schaftwand 203, welche ein Lumen 237 umschließt. Am äußeren distalen Ende des Katheters ist eine Elektrode 235 angebracht, welche über einen Zuleitungsdraht 238 elektrisch mit dem proximalen Ende des Ablationskatheters 230 verbunden ist. Der Zuleitungsdraht 238 verläuft in dem Lumen 237. Die Schaftwand 203 ist fluiddicht ausgebildet. Die Elektrode 205 weist einen Elektrodentemperatursensor 252 auf, welcher mit einer entlang der Katheterlängsachse zum proximalen Ende des Katheters verlaufenden elektrischen Verbindungsleitung 250 verbunden ist, welche in dem Lumen 237 angeordnet ist. Der Katheter umfasst auch einen Drahttemperatursensor 255, welcher im Bereich des distalen Endes des Katheters 230 in dem Lumen 237 angeordnet ist und mit einer zum proximalen Ende des Katheters in dem ersten Lumen entlang der Katheterlängsachse verlaufenden elektrischen Verbindungsleitung 257 verbunden ist. Die Elektrode 235 weist Öffnungen 234 auf, welche mit dem Lumen 237 fluidverbunden sind, so dass Fluid durch die Öffnungen nach aussen strömen kann.

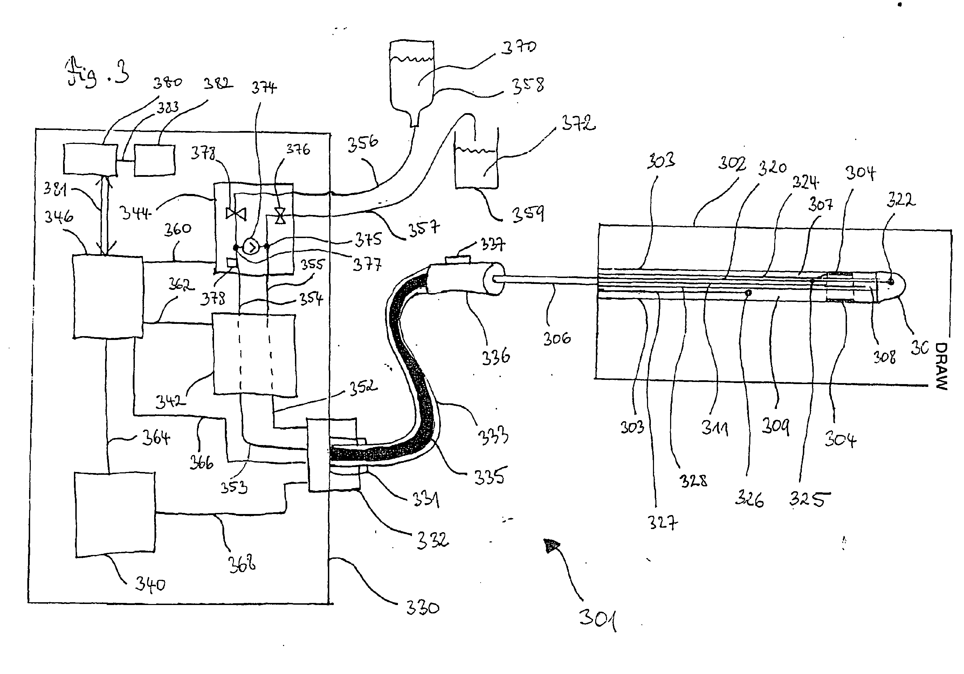

Figur 3 zeigt eine Anordnung 301 mit einem Versorgungsgerät 330 und einem Ablationskatheter, welcher über eine Buchse 332 mit dem Versorgungsgerät 330 verbunden ist.

Der Ablationskatheter umfasst einen Katheterschaft 306, welcher in einen Handgriff 336 mündet, wobei der Handgriff 336 mit einem flexiblen Schlauch 333 verbunden ist. An den flexiblen Schlauch 333 ist am Ende ein Stecker 331 angebracht, welcher mit der Buchse 332 verbindbar ist.

Das distale Ende des Katheterschaftes 306 ist in Figur 3 in einem Vergrößerungsfenster 302 dargestellt. Der Katheterschaft 306 weist eine Schaftwand 303 auf, welche ein erstes Lumen 309 und ein zweites Lumen 307 umschließt. Das erste Lumen 309 und das zweite Lumen 307 sind durch eine Trennwand 311 voneinander getrennt. Die Anordnung der Schaftwand 303, des ersten Lumens 309, des zweiten Lumens 307 und der Trennwand 311 ist analog dem in Figur 1b dargestellten Querschnitt ausgeführt. Das erste Lumen 309, das zweite Lumen 307 und die Trennwand 311 sind vom distalen Ende des Katheterschaftes 306 bis zum proximalen Ende des Katheterschaftes 306 ausgebildet. Der Katheterschaft weist einen Steuerdraht 328 auf, welcher am distalen Ende des Katheterschaftes befestigt ist und entlang der Katheterlängsachse in dem ersten Lumen 309 bis in den Handgriff 336 führt, wobei in dem Handgriff der Steuerdraht 328 mit dem Schieber 337 verbunden ist. Ein Betätigen des Schiebers 337 bewirkt eine Verkrümmung des distalen Endes des Katheters mit einer Richtungskomponente orthogonal zur Katheterlängsachse. Am äußeren distalen Ende des Katheterschaftes ist ein Ablationselektrode 305 angebracht. Die Ablationselektrode ist als bipolare Elektrode ausgebildet und umfasst dazu eine Gegenelektrode 304, welche als Ringelektrode am distalen Ende des Katheterschaftes ausgebildet ist. Der Katheterschaft weist einen Elektrodentemperatursensor 322 auf, welcher an der Hochfrequenzelektrode 305 angebracht ist und mit einer Temperatursensorleitung 320 verbunden ist, welche durch das zweite Lumen 307 entlang der Katheterlängsachse in den Handgriff 336 mündet. Ein Drahttemperatursensor 325 ist im Bereich des distalen Endes in dem zweiten Lumen 307 angeordnet und ist mit der Temperatursensorleitung 320 verbunden, welche als zweikanalige Temperatursensorleitung ausgebildet ist. Ein Fluidtemperatursensor ist im Bereich des distalen Endes des Katheters in dem ersten Lumen angeordnet und ist mit einer elektrischen Fluidtemperatursensorleitung 327 verbunden, welche entlang der Katheterlängsachse in dem ersten Lumen geführt ist und in den Handgriff 336 mündet. Die Hochfrequenzelektrode 305 und die Gegenelektrode 304 sind mit einer zweikanaligen Hochfrequenzleitung 324 verbunden, welche in dem zweiten Lumen entlang der Elektrodenleitungslängsachse geführt ist und in den Handgriff 336 mündet. Das erste Lumen 309, die Fluidtemperatursensorleitung 327 und die Temperatursensorleitung 320 sind in dem Handgriff 336 zu einem Leitungsbündel 335 zusammengeführt, welches in den Stecker 331 mündet. In die Buchse 332 des Versorgungsgerätes 330 münden eine erste Fluidleitung 353, eine zweite Fluidleitung 352, eine elektrische Hochfrequenzleitung 368 und eine dreikanalige Temperatursensorleitung 366. Im Falle der Kopplung des Steckers 331 mit. der Buchse 332 ist die erste Fluidleitung 353 mit dem ersten Lumen 309 fluidverbunden, die zweite Fluidleitung 352 mit dem zweiten Lumen 307 fluidverbunden, die elektrische Hochfrequenzleitung 368 mit der Hochfrequenzleitung 324 elektrisch verbunden und die dreikanalige Temperatursensorleitung 366 mit der Fluidtemperatursensorleitung 327 und der Temperatursensorleitung 320 elektrisch verbunden.

Das Versorgungsgerät 330 umfasst eine Fluidzufuhreinheit 344, welche eine Zirkulationspumpe 374 aufweist, welche an Fluidleitungen 355 und 354 angeschlossen ist. Das Versorgungsgerät 330 weist auch eine steuerbare Kühleinheit 342 mit einem Steuereingang auf, welche einen Thermostat zur Einhaltung einer vorbestimmten Fluidtemperatur aufweist. Die Kühleinheit 342 ist an die Fluidleitungen 354, 355, 352 und 353 angeschlossen, wobei die Fluidleitung 355 über die Kühleinheit 342 mit der zweiten Fluidleitung 352 fluidverbunden ist, und die Fluidleitung 354 über die Kühleinheit 342 mit der ersten Fluidleitung 353 fluidverbunden ist. Die Kühleinheit 342 ist bevorzugt als Peltier-Kühleinheit ausgebildet und umfasst dazu wenigstens ein Peltierelement. Die Anordnung 301 umfasst auch einen Fluidzufuhrbehälter 358, welcher über eine Fluidzufuhrleitung 356 mit der Fluidzufuhreinheit 344 verbunden ist und dort über ein in der Fluidzufuhreinheit enthaltenes Einlassventil 378 mit dem Pumpeneingang 377 verbunden ist, an welchen auch die Fluidleitung 354 angeschlossen ist. Die Anordnung 301 umfasst auch einen Fluidabfuhrbehälter 359, in welchen eine Fluidabfuhrleitung 357 mündet, welche über ein in der Fluidzufuhreinheit 344 enthaltenes Überdruckventil 376 mit dem Zirkulationspumpenausgang 375 im Falle der Öffnung des Überdruckventils 376 fluidverbunden ist. Der Zirkulationspumpenausgang 375 ist an die Fluidleitung 355 angeschlossen. Die Ventile 376 und 378 sowie die Pumpe 374 sind steuerbar ausgebildet. Dazu weist die Fluidzufuhreinheit 344 einen Steuereingang auf und ist über eine Steuerleitung 360 mit dem Steuerausgang einer Temperaturkontroll- und Steuereinheit 346 verbunden. Die Fluidzufuhreinheit 344 ist ausgebildet, aufgrund der Steuerinformation eines über die Steuerleitung 360 empfangenen Steuersignales wahlweise das Pumpvolumen der Zirkulationspumpe 374 einzustellen oder die Ventile 378 und 376 zu öffnen oder zu schließen. Die Temperaturkontroll- und Steuereinheit 346 ist ausgangsseitig über eine elektrische Verbindungsleitung 362 zur Steuerung der Kühleinheit 342 mit deren Steuereingang verbunden. Die Kühleinheit 342 ist ausgebildet, aufgrund eines über die Steuerleitung empfangenen Steuersignales ein durch die Kühleinheit 342 fließendes Fluid zu kühlen. Die Kühleinheit 342 kann auch ausgebildet sein, eine dem Steuersignal entprechende Kühlleistung oder Fluidtemperatur einstellen. Dazu kann die Kühleinheit 342 einen Temperaturregler umfassen. Die Temperaturkontroll- und Steuereinheit ist über eine dreikanalige Temperatursensorleitung 366 mit den im Katheterschaft enthaltenen Temperatursensoren 325, 326 und 322 wirkverbunden.

Der Fluidtemperatursensor 326 kann alternativ zur Anordnung in dem ersten Lumen 309 des Katheterschaftes 306 auch in dem Handgriff 336 angeordnet sein und ist dort ebenfalls in dem ersten Lumen 309 angeordnet oder mit dem ersten Lumen 309 wärmeleitfähig verbunden.

Das Versorgungsgerät 330 umfasst auch einen Hochfrequenzgenerator 340, welcher über eine elektrische Hochfrequenzleitung 368 und über eine elektrische Hochfrequenzleitung 324 mit der Hochfrequenzelektrode 305 und der Gegenelektrode 304 verbunden ist. Die elektrischen Hochfrequenzleitungen 368 und 324 sind dazu zweikanalig ausgebildet. Der Hochfrequenzgenerator 340 ist über eine Hochfrequenzsteuerleitung 364 mit der Temperaturkontroll- und Steuereinheit 346 verbunden. Die Temperaturkontroll- und Steuereinheit 346 ist über einen bidirektionalen Datenbus 381 an eine Eingabeeinheit 380 angeschlossen, wobei die Eingabeeinheit über eine Verbindungsleitung 383 mit einem Display 382 verbunden ist.

Im Folgenden soll nun die Funktionsweise der Anordnung näher erläutert werden:

Über die Eingabeeinheit 380, welche beispielsweise ein Tastenfeld umfasst, können Betriebsparameter zum Betreiben des Ablationskatheters in die Temperaturkontrollund Steuereinheit 346 eingegeben werden. Diese sind beispielsweise das Ein- und Ausschalten einer Hochfrequenzleistung oder das Vorwählen einer vorbestimmten Hochfrequenzleistung, welche über die Hochfrequenzelektrode 305 abgegeben werden soll, oder das Vorwählen einer gewünschten Ablationselektrodentemperatur. Die Temperaturkontroll- und Steuereinheit 346, welche beispielsweise einen Mikroprozessor aufweist, ist ausgebildet, entsprechend der Vorgabe durch die Eingabeeinheit 380 ein dem Eingabewert entsprechendes Signal zur Erzeugung einer dem Eingabewert entsprechenden Hochfrequenzleistung über die Hochfrequenzsteuerleitung 364 an den Hochfrequenzgenerator 340 zu senden. Der Hochfrequenzgenerator 340 ist ausgebildet, aufgrund des über die Hochfrequenzsteuerleitung 364 empfangenen Steuersignales eine dem Eingabewert entsprechende Hochfrequenzleistung über die elektrische Hochfrequenzleitung 368 und die mit dieser wirkverbundenen Hochfrequenzelektrode 305 abzugeben. Die Temperatursensoren 325, 326 und 322 sind ausgebildet, ein die erfasste Temperatur repräsentierendes Temperatursignal zu erzeugen. Die von den Temperatursensoren jeweils erzeugten Temperatursignale stehen über die jeweils mit den Temperatursensoren verbundenen Temperatursensorleitungen 327 und 320, sowie über die dreikanalige Temperatursensorleitung 366 der Temperaturkontroll- und Steuereinheit 346 zur Verfügung.

Die Temperaturkontroll- und Steuereinheit 346 ist ausgebildet, die über die dreikanalige Temperatursensorleitung 366 empfangenen Temperatursignale auszuwerten, und das Pumpvolumen der Zirkulationspumpe 374 über die Verbindungsleitung 360, die Kühlleistung der Kühleinheit 342 über die Verbindungsleitung 362 sowie die Hochfrequenzleistung über die Hochfrequenzsteuerleitung 364 gemäß einem in der Temperaturkontroll- und Steuereinheit 346 vorgegebenen Regelalgorithmus zu steuern und dazu entsprechende Steuersignale zu erzeugen. Die Temperaturkontroll- und Steuereinheit 346 kann Statusinformationen entsprechende Signale über den bidirektionalen Datenbus 381 senden, die Eingabeeinheit 380 und die Verbindungsleitung 383 an das Display 382 senden, welches diese Statusinformationen anzeigen kann. Die Statusinformationen können beispielsweise die Temperatur der Hochfrequenzelektrode 305, die von dem Fluidtemperatursensor 326 erfasste Fluidtemperatur des Fluides 370, welches sich in dem ersten Lumen 309 befindet, sowie die von dem Drahttemperatursensor 325 erfasste Fluidtemperatur des Fluides 370, welches sich in dem zweiten Lumen 307 befindet sein. Die Temperaturkontroll- und Steuereinheit 346 ist auch ausgebildet, ein über die Verbindungsleitung 366 empfangenes Signal eines Fluidsensors auszuwerten, welcher in der Fluidzufuhreinheit angebracht ist und den Fluidfüllzustand und/oder den Fluiddruck in dem durch die Fluidleitungen und die Lumina gebildeten Fluidkreislauf zu überwachen und das Einlassventil 378 zum Einlassen eines Fluides 370 über die Fluidzufuhrleitung 365 in den Fluidkreislauf entsprechend zu steuern. Bei einem durch die Erwärmung des Katheterschaftes erzeugten Überdruck in dem Fluidkreislauf ist das Überdruckventil 376 ausgebildet, bei einem vorbestimmten Überdruck zu öffnen und somit überschüssiges Fluid 372 über die Fluidabfuhrleitung 357 in den Fluidabfuhrbehälter 359 abzugeben.

Die in der Figurenbeschreibung aufgezeigten Merkmale können auch für sich genommen unabhängig von den anderen in diesem Zusammenhang aufgezeigten Merkmalen an einem Katheter oder einer Anordnung mit einem Katheter verwirklicht sein.

## Patentansprüche

1. Ablationskatheter (151) zur intravaskulären Applikation, mit einem proximalen und einem distalen Ende und einer Katheterlängsachse, mit
einem sich entlang der Katheterlängsachse von dem proximalen bis zu dem distalen Ende erstreckenden Schaft mit einer fluiddichten Schaftwand (152), welche ein Lumen (153, 207) ausbildet,
mit mindestens einer Ablationselektrode (205, 235, 304, 305) am distalen Ende des Schaftes,
wobei das Lumen ein erstes Lumen (158) und ein zweites Lumen (154, 207) umfasst, welche jeweils ausgebildet sind, ein Fluid zu führen und sich vom proximalen bis zumindest in die Nähe des distalen Endes des Katheters erstrecken und von dem Schaft umschlossen sind,
mindestens einem elektrisch leitfähigen Draht (156, 224, 254 311), welcher sich vom proximalen bis zumindest in die Nähe des distalen Endes des Katheters entlang der Katheterlängsachse erstreckt und sich auf mindestens einer zum Einführen in einen Körper vorgesehenen Abschnittslänge des Katheters im zweiten Lumen befindet, wobei zwischen einem in dem zweiten Lumen befindlichen Fluid und dem elektrisch leitfähigen Draht ein geringerer Wärmeübergangswiderstand herrscht, als zwischen dem Draht und der Schaftwand, so dass in dem Draht entstehende Wärme an das im Lumen fließende Fluid wirksam abgeführt werden kann,
und
mindestens einem Temperatursensor (222, 224, 226, 252, 255, 322, 326), welcher angeordnet ist, eine Temperatur zu erfassen, welche im Falle einer Erwärmung des Drahtes zumindest überwiegend von der Erwärmung des Drahtes abhängt,
**dadurch gekennzeichnet, dass**
das Lumen ein drittes Lumen (159) umfasst, welches ausgebildet ist, ein Fluid zu führen und sich vom proximalen bis zumindest in die Nähe des distalen Endes des Katheters erstreckt und von dem Schaft umschlossen ist
und das distale Ende des Ablationskatheters ausgebildet ist, ausschließlich ein in einem ersten Lumen (158) strömendes Fluid der mindestens einen Ablationselektrode zur Kühlung zuzuführen und ein in dem ersten Lumen zum distalen Ende strömendes Fluid zusammen mit dem Fluid zur Kühlung des mindestens einen Drahtes (156), welches durch das zweite Lumen (154) strömt, in dem dritten Lumen (159) in Richtung proximales Ende zurückzuführen.

2. Ablationskatheter nach Anspruch 1, **dadurch gekennzeichnet, dass** der Katheter wenigstens einen Temperatursensor (222, 224, 226, 252, 255) zur Erfassung der Temperatur des Fluides oder des Drahtes oder der Schaftwand aufweist.

3. Ablationskatheter nach einem der vorhergehenden Ansprüche, **dadurch gekennzeichnet, dass** der Draht eine elektrische Zuleitung ist.

4. Ablationskatheter nach einem der vorhergehenden Ansprüche, **dadurch gekennzeichnet, dass** der Draht ein Steuerdraht ist, welcher in dem inneren Lumen in Richtung der Katheterlängsachse verschieblich angeordnet ist.

5. Ablationskatheter nach einem der vorhergehenden Ansprüche, **dadurch gekennzeichnet, dass** der Katheter entlang der Katheterlängsachse mehrere Temperatursensoren (222, 224, 226, 252, 255) aufweist, welche derart angeordnet sind, die Temperatur eines Drahtes und/oder der Schaftwand und/oder eines Lumens zu erfassen.

6. Ablationskatheter nach einem der vorhergehenden Ansprüche, **dadurch gekennzeichnet, dass** der Katheter zusätzlich wenigstens eine Sensingelektrode aufweist und so auch zum Mapping geeignet ist.

7. Ablationskatheter nach einem der vorhergehenden Ansprüche, **dadurch gekennzeichnet, dass** der Katheter wenigstens einen Temperatursensor (326) aufweist, welcher entlang der Katheterlängsachse im Bereich des zur intrakorporalen Invasion vorgesehenen Abschnittes des Katheterschaftes entfernt von gegebenenfalls vorgesehenen Ablationselektroden angeordnet ist.

8. Anordnung zur intravaskulären Invasion, umfassend einen Ablationskatheter nach einem der vorhergehenden Ansprüche und
eine Fluidzufuhreinheit (344), welche mit wenigstens einem Lumen des Katheters fluidverbunden und ausgebildet ist, einen Fluidstrom in das Lumen zu erzeugen.

9. Anordnung nach Anspruch 8, **dadurch gekennzeichnet, dass** die Fluidzufuhreinheit eine steuerbare Pumpeinheit (374) enthält, welche wenigstens mit einem Lumen des Ablationskatheters fluidverbunden und ausgebildet ist, in Abhängigkeit von einem Fluidsteuersignal ein Fluid in das Lumen zu pumpen.

10. Anordnung nach einem der Ansprüche 8 oder 9, **dadurch gekennzeichnet, dass** die Anordnung eine Kühleinheit (342) umfasst, welche mit wenigstens einem Lumen des Ablationskatheters fluidverbunden ist und die Kühleinheit ausgebildet ist, in Abhängigkeit von einem Kühlsteuersignal einem Fluid Wärme zu entziehen.

11. Anordnung nach Anspruch 9 oder 10, **dadurch gekennzeichnet, dass** die Anordnung eine Temperaturkontrolleinheit (346) aufweist, die mit wenigstens einem Temperatursensor (322, 326), der Pumpeinheit (374) oder der Kühleinheit (342) oder beiden wirkverbunden ist, wobei der Temperatursensor ausgebildet ist, ein eine Temperatur repräsentierendes Temperatursignal zu erzeugen und die Temperaturkontrolleinheit ausgebildet ist, ein von dem Temperatursensor erzeugtes Temperatursignal auszuwerten, und die Pumpeinheit oder die Kühleinheit oder beide gemäß einem in der Temperaturkontrolleinheit vorgegebenen Regelalgorithmus zu steuern und dazu entsprechende Steuersignale zu erzeugen.

## Claims

1. An ablation catheter (151) for intravascular application, with a proximal end and a distal end and a catheter longitudinal axis, comprising
a shaft extending along the catheter longitudinal axis from the proximal end to the distal end and having a fluid-tight shaft wall (152), which forms a lumen (153, 207),
comprising at least one ablation electrode (205, 235, 304, 305) at the distal end of the shaft,
wherein the lumen comprises a first lumen (158) and a second lumen (154, 207), which are each designed to guide a fluid and extend from the proximal end at least into the vicinity of the distal end of the catheter and are enclosed by the shaft,
at least one electrically conductive wire (156, 224, 254, 311), which extends from the proximal end at least into the vicinity of the distal end of the catheter along the catheter longitudinal axis and is located in the second lumen on at least one portion of the length of the catheter provided for insertion into a body, wherein a lower level of heat transmission resistance prevails between a fluid located in the second lumen and the electrically conductive wire than between the wire and the shaft wall so that heat produced in the wire can be effectively dissipated to the fluid flowing in the lumen,
and
at least one temperature sensor (222, 224, 226, 252, 255, 322, 326), which is arranged to detect a temperature which, when the wire is heated, depends at least predominantly on the heating of the wire,
**characterised in that**
the lumen comprises a third lumen (159), which is designed to guide a fluid and extends from the proximal end at least into the vicinity of the distal end of the catheter and is enclosed by the shaft
and the distal end of the ablation catheter is designed to feed exclusively a fluid flowing in a first lumen (158) to the at least one ablation electrode for cooling, and to return, in the third lumen (159) in the direction of the proximal end, a fluid flowing in the first lumen to the distal end together with the fluid for cooling the at least one wire (156), which flows through the second lumen (154).

2. The ablation catheter according to Claim 1, **characterised in that** the catheter has at least one temperature sensor (222, 224, 226, 252, 255) for detecting the temperature of the fluid or of the wire or of the shaft wall.

3. The ablation catheter according to one of the preceding claims, **characterised in that** the wire is an electric feed line.

4. The ablation catheter according to one of the preceding claims, **characterised in that** the wire is a control wire, which is arranged in the inner lumen displaceably in the direction of the catheter longitudinal axis.

5. The ablation catheter according to one of the preceding claims, **characterised in that** the catheter has a plurality of temperature sensors (222, 224, 226, 252, 255) along the catheter longitudinal axis, which are arranged in such a way to detect the temperature of a wire and/or of the shaft wall and/or of a lumen.

6. The ablation catheter according to one of the preceding claims, **characterised in that** the catheter additionally has at least one sensing electrode and is thus also suitable for mapping.

7. The ablation catheter according to one of the preceding claims, **characterised in that** the catheter has at least one temperature sensor (326), which is arranged along the catheter longitudinal axis in the region of the portion of the catheter shaft provided for intracorporeal invasion, removed from ablation electrodes possibly provided.

8. An arrangement for intravascular invasion, comprising an ablation catheter according to one of the preceding claims and
a fluid feed unit (344), which is in fluid communication with at least one lumen of the catheter and is designed to generate a fluid flow into the lumen.

9. The arrangement according to Claim 8, **characterised in that** the fluid feed unit contains a controllable pump unit (374), which is in fluid communication at least with one lumen of the ablation catheter and which is designed to pump a fluid into the lumen according to a fluid control signal.

10. The arrangement as claimed in one of Claims 8 or 9, **characterised in that** the arrangement comprises a cooling unit (342), which is in fluid communication with at least one lumen of the ablation catheter, and the cooling unit is designed to take heat from a fluid according to a cooling control signal.

11. The arrangement according to Claim 9 or 10, **characterised in that** the arrangement has a temperature monitoring unit (346), which is operatively connected to at least one temperature sensor (322, 326), the pump unit (374) or the cooling unit (342) or both, wherein the temperature sensor is designed to generate a temperature signal representative of a temperature and the temperature monitoring unit is designed to evaluate a temperature signal generated by the temperature sensor and to control the pump unit or the cooling unit or both in accordance with a regulating algorithm predetermined in the temperature monitoring unit and to generate control signals corresponding thereto.

## Revendications

1. Cathéter d'ablation (151) destiné à une application intra-vasculaire, avec une extrémité proximale et une extrémité distale et un axe longitudinal de cathéter, avec
- une tige s'étendant le long de l'axe longitudinal du cathéter, de l'extrémité proximale jusqu'à l'extrémité distale, avec une paroi de tige étanche aux fluides (152), formant un canal (153, 207),
- avec au moins une électrode d'ablation (205, 235, 304, 305) à l'extrémité distale de la tige,
dans lequel le canal comprend un premier canal (158) et un deuxième canal (154, 207), respectivement conçus pour guider un fluide et s'étendant à partir de l'extrémité proximale, au moins jusqu'à proximité de l'extrémité distale du cathéter, tout en étant entourés par la tige,
- au moins un fil électriquement conducteur (156, 224, 254, 311) s'étendant à partir de l'extrémité proximale au moins jusqu'à proximité de l'extrémité distale du cathéter, le long de l'axe longitudinal du cathéter, et se trouvant sur au moins une longueur de segment du cathéter dans le deuxième canal, prévue pour l'introduction dans un corps, dans lequel la résistance au transfert de chaleur est plus faible entre un fluide situé dans le deuxième canal et le fil électriquement conducteur qu'entre le fil et la paroi de tige, de sorte qu'une chaleur produite dans le fil peut être efficacement transmise au fluide s'écoulant dans le canal,
et
- au moins un capteur de température (222, 224, 226, 252, 255, 322, 326) agencé de manière à détecter une température dépendant au moins majoritairement de l'échauffement du fil, dans le cas d'un échauffement du fil,
**caractérisé en ce que**
- le canal comprend un troisième canal (159) conçu pour guider un fluide et s'étendant à partir de l'extrémité proximale au moins jusqu'à proximité de l'extrémité distale du cathéter, tout en étant entouré par la tige,
- et l'extrémité distale du cathéter d'ablation est conçue pour alimenter un fluide s'écoulant exclusivement dans le premier canal (158), vers l'au moins une électrode d'ablation, pour le refroidissement, et pour renvoyer un fluide s'écoulant vers l'extrémité distale dans le premier canal, ensemble avec le fluide destiné au refroidissement de l'au moins un fil (156), lequel s'écoule à travers le deuxième canal (154), vers le troisième canal (159), en direction de l'extrémité proximale.

2. Cathéter d'ablation selon la revendication 1, **caractérisé en ce que** le cathéter comporte au moins un capteur de température (222, 224, 226, 252, 255) pour la détection de la température du fluide ou du fil ou de la paroi de tige.

3. Cathéter d'ablation selon l'une des revendications précédentes, **caractérisé en ce que** le fil est une ligne d'alimentation électrique.

4. Cathéter d'ablation selon l'une des revendications précédentes, **caractérisé en ce que** le fil est un fil de commande agencé dans le canal interne, de façon déplaçable dans la direction de l'axe longitudinal du cathéter.

5. Cathéter d'ablation selon l'une des revendications précédentes, **caractérisé en ce que** le cathéter comporte plusieurs capteurs de température (222, 224, 226, 252, 255) le long de l'axe longitudinal du cathéter, agencés de manière à détecter la température d'un fil et/ou de la paroi de tige et/ou d'un canal.

6. Cathéter d'ablation selon l'une des revendications précédentes, **caractérisé en ce que** le cathéter comporte en outre au moins une électrode de détection, le rendant ainsi adapté pour la cartographie.

7. Cathéter d'ablation selon l'une des revendications précédentes, **caractérisé en ce que** le cathéter comporte au moins un capteur de température (326) agencé le long de l'axe longitudinal du cathéter, dans la région du segment de tige de cathéter prévu pour l'invasion intracorporelle, à distance des éventuelles électrodes d'ablation.

8. Agencement pour l'invasion intra-vasculaire, comprenant un cathéter d'ablation selon l'une des revendications précédentes, et
une unité d'alimentation en fluide (344) en communication fluidique avec au moins un canal du cathéter, conçue pour produire un flux de fluide dans le canal.

9. Agencement selon la revendication 8, **caractérisé en ce que** l'unité d'alimentation en fluide comprend une unité de pompe réglable fluidiquement reliée à au moins un canal du cathéter d'ablation, et conçue pour pomper un fluide dans le canal en fonction d'un signal de commande de fluide.

10. Agencement selon l'une des revendications 8 ou 9, **caractérisé en ce que** l'agencement comprend une unité de refroidissement (342) fluidiquement reliée à au moins un canal du cathéter d'ablation, l'unité de refroidissement étant conçue pour extraire de la chaleur d'un fluide en fonction d'un signal de commande de refroidissement.

11. Agencement selon la revendication 9 ou 10, **caractérisé en ce que** l'agencement comporte une unité de contrôle de température (346) fonctionnellement reliée au moins à un capteur de température (322, 326), l'unité de pompe (374) ou l'unité de refroidissement (342) ou aux deux, le capteur de température étant conçu pour produire un signal de température représentant une température, et l'unité de contrôle de température étant conçue pour évaluer un signal de température produit par le capteur de température, et pour commander l'unité de pompe ou l'unité de refroidissement ou les deux, en fonction d'un algorithme de contrôle prédéterminé dans l'unité de contrôle de la température, et pour produire à cette fin des signaux de commande correspondants.
